Europäisches Patentamt

European Patent Office

Office européen des brevets

(11) Veröffentlichungsnummer : **0 160 749**
**B1**

(12) EUROPÄISCHE PATENTSCHRIFT

(45) Veröffentlichungstag der Patentschrift :
07.06.89

(51) Int. Cl.⁴ : **A 61 B 6/00**

(21) Anmeldenummer : **84116150.8**

(22) Anmeldetag : **21.12.84**

(54) Röntgenuntersuchungsgerät.

(30) Priorität : 09.04.84 DE 3413348

(43) Veröffentlichungstag der Anmeldung :
13.11.85 Patentblatt 85/46

(45) Bekanntmachung des Hinweises auf die Patenterteilung : 07.06.89 Patentblatt 89/23

(84) Benannte Vertragsstaaten :
DE FR GB IT NL

(56) Entgegenhaltungen :
DE-A- 1 959 358
DE-A- 2 108 657
FR-A- 1 155 980
FR-A- 2 115 423
FR-A- 2 291 733
FR-A- 2 342 704
FR-A- 2 405 695
GB-A- 2 056 830
GB-A- 2 098 440

(73) Patentinhaber : Siemens Aktiengesellschaft Berlin
und München
Wittelsbacherplatz 2
D-8000 München 2 (DE)

(72) Erfinder : Barud, Sigvard, Dipl.-Ing.
Roedluvevaegen 73
S-17 570 Jaerfaella (SE)

## Beschreibung

Die Erfindung betrifft ein Röntgenuntersuchungsgerät mit einem Stativ und einer Halterung, die im Raum bzw. gegeneinander derart verstellbar sind, dass ein an der Halterung befestigter gebogener Träger, an dessen einem Ende eine Strahlungsquelle und an dessen anderem Ende ein Bildschichtträger aufeinander ausgerichtet befestigt sind, dreidimensional einstellbar ist, dass der Träger um eine erste Welle drehbar ist und um eine weitere, horizontal und senkrecht zur ersten Welle am vorderen Ende der Halterung liegenden Welle schwenkbar gelagert ist.

Durch die deutschen Offenlegungsschriften 2 608 461 und 3 217 478 und durch die FR-A-1 155 980 sind Röntgenuntersuchungsgeräte bekannt, bei denen eine Röntgenröhre und ein Röntgenbildverstärker an einander gegenüberliegenden Enden eines halbkreisförmig gebogenen Trägers befestigt sind. Der halbkreisförmig gebogene Träger ist in einer Schiene längs seines Umfanges verschiebbar. Damit der Träger in dieser einwandfrei verschoben werden kann, müssen beide mit hoher Präzision gefertigt werden. Dies ist aufwendig und verteuert das Röntgenuntersuchungsgerät sehr.

Der Erfindung liegt die Aufgabe zugrunde, ein Röntgenuntersuchungsgerät der eingangs genannten Art zu schaffen, das im Aufbau einfacher und daher billiger ist als die konventionellen Röntgenuntersuchungsgeräte.

Diese Aufgabe ist erfindungsgemäss dadurch gelöst, dass der Träger unverschiebbar an der Halterung befestigt ist, dass für jede Verstell-, Dreh- oder Schwenkbewegung ein Antrieb und ein Lagesensor vorgesehen sind. Durch den damit möglichen Bewegungsablauf der verschiedenen Teile des Röntgenuntersuchungsgerätes ist es nicht mehr notwendig, einen teuren, halbkreisförmig gebogenen Träger zu verwenden. Der Träger kann nunmehr U- oder V-förmig ausgebildet sein, was herstellungsmässig wesentlich einfacher ist und dadurch das Gerät billiger macht. Ferner wird keine gebogene, dem Träger angepasste Schiene benötigt.

Bei einem gemäss der Erfindung ausgestalteten Röntgenuntersuchungsgerät wird vorgeschlagen, dass die Länge der Halterung einstellbar ist. Dadurch ist erreicht, dass das Stativ lediglich in einer Ebene bewegbar sein muss. Wenn die Halterung darüberhinaus gegen das Stativ verschieb- oder schwenkbar ist, reduziert sich die notwendige Bewegung des Statives auf eine Richtung.

Im Hinblick auf eine konstruktiv einfache Einstellung der Länge der Halterung empfiehlt es sich, diese als Teleskop auszubilden. Alternativ kann die Halterung als ein zweiteiliger Gelenkarm ausgebildet sein.

In einer zweckmässigen Ausgestaltung der Erfindung wird vorgeschlagen, dass die Halterung an dem Stativ um eine Welle in vertikaler Richtung schwenkbar gelagert ist. Dadurch ist erreicht, dass das Stativ niedrig gehalten werden kann.

Damit der Träger unbehindert in einem grossen Winkelbereich schwenkbar ist, ist in einer konstruktiv vorteilhaften Ausgestaltung der Erfindung vorgesehen, dass der Träger seitlich am vorderen Ende der Halterung angebracht ist.

In einer vorteilhaften Weiterbildung der Erfindung wird vorgeschlagen, dass die Signale der Lagesensoren auf eine Regeleinrichtung gegeben sind, die bei vorgegebenem Isozentrum bei einer Lageänderung in einer Richtung Regelgrössen für die anderen Antriebe derart ermittelt, dass durch deren Betätigung wieder das Isozentrum eingestellt wird. Dadurch ist erreicht, dass der Träger derart gesteuert werden kann, dass unabhängig vom Bewegungsablauf der Röntgenröhre bzw. des Bildverstärkers das Isozentrum immer beibehalten werden kann.

Die Erfindung ist nachfolgend anhand von mehreren Ausführungsbeispielen in Verbindung mit den Zeichnungen näher erläutert. Es zeigen :

Fig. 1 eine Seitenansicht eines Röntgenuntersuchungsgerätes nach der Erfindung,

Fig. 2 eine schematische Darstellung einer Steuervorrichtung für ein Röntgenuntersuchungsgerät nach Fig. 1, und

Fig. 3 bis 5 weitere Ausführungsformen eines Röntgenuntersuchungsgerätes nach der Erfindung.

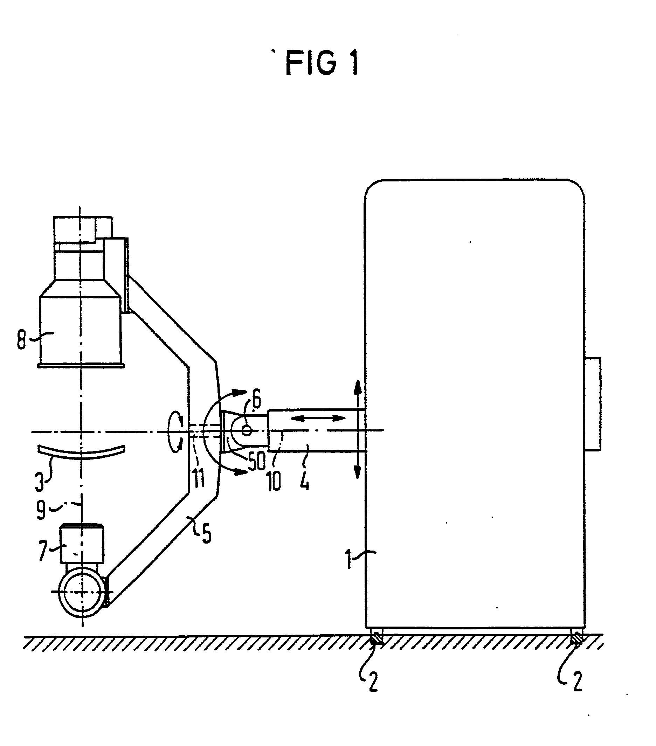

Fig. 1 zeigt ein Röntgenuntersuchungsgerät mit einem Stativ 1, der auf Bodenschienen 2 längsverschieblich und parallel zu einem Patientenlagerungstisch 3 geführt werden kann.

An dem Stativ 1 ist eine Halterung 4 für einen U- oder V-förmig gebogenen Träger 5 vertikal einstellbar gelagert. Der Träger ist um eine senkrecht zur Längsrichtung der Halterung 4 an derem vorderen Ende angebrachten Welle 6 über einen Teil 50 schwenkbar gelagert. Der Träger 5 kann auch direkt an der Welle 6 angebracht sein. Der Träger 5 ist vorzugsweise seitlich am vorderen Ende der Halterung 4 angebracht. An dem einem Ende des Trägers 5 ist ein Röntgenröhre 7 und an dem anderen Ende ist ein Bildschichtträger 8 befestigt. Die Röntgenröhre 7 ist mit ihrem Zentralstrahl 9 auf dem Bildschichtträger 8 zentriert. Der Träger ist ferner am eine Welle 11 drehbar gelagert, die in Verlängerung der Achse 10 der Halterung 4 liegt. Die Halterung 4 ist auch als Teleskop ausgebildet, so dass deren Länge in horizontaler Richtung einstellbar ist.

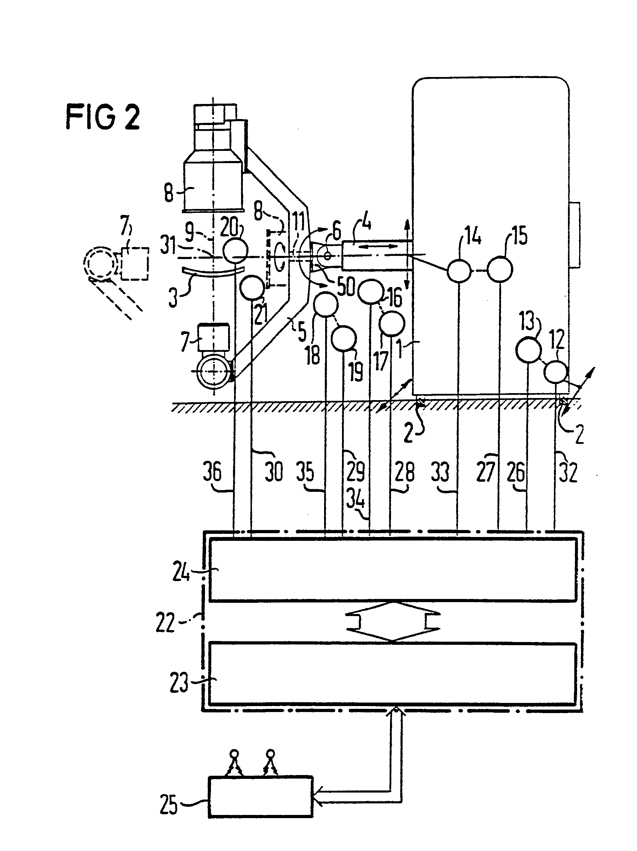

In Fig. 2 ist dargestellt, das für jede Verstell-, Dreh- oder Schwenkbewegung der Geräteteile ein Antrieb und ein Lagesensor vorgesehen sind. So wird das Stativ 1 mittels eines Motors 12 und eines Lagesensors 13 gesteuert. Die Höhenverstellung der Halterung 4 erfolgt mittels eines Motors 14 und eines Lagesensors 15 und die Länge der teleskopartigen Halterung 4 wird durch einen Motor 16 und einen Lagesensor 17 eingestellt. Ein weiterer Motor 18 und ein Lagesensor 19 steuern die Schwenkbewegung des Trägers 5 um die Welle 6. Schliesslich ist ein Motor 20 und ein Lagesensor 21 für die Drehung des Trägers 4 um die Welle 11 vorgesehen.

Als Regeleinrichtung 22 für die Motoren 12, 14, 16, 18, 20 und die Lagesensoren 13, 15, 17, 19, 21 dient ein Computer 23 und eine Steuervorrichtung 24. An der Regeleinrichtung 22 ist ein Steuerpult 25 angeschlossen, wodurch die Lage der Röntgenröhre 7 bzw. des Bildschichtträgers 8 gesteuert werden. Eine derartige Regeleinrichtung mit Steuerpult ist an sich grundsätzlich durch die US-PS 4 019 059 bekannt und wird daher nicht näher beschrieben.

Wenn eine Lageänderung der Röntgenröhre 7 bzw. des Bildschirmträgers 8 vorgenommen werden soll, werden ausgewählte Motoren 12, 14, 16, 18, 20 mittels des Steuerpults 25 angesteuert. Die Signale der Lagesensoren 13, 15, 17, 19, 21 werden dabei über Verbindungen 26 bis 30 der Regeleinrichtung 22 zugeführt. Die Regeleinrichtung 22 führt dann bei einem vorgegebenen Isozentrum 31 über die Verbindung 32 bis 36 den übrigen Motoren 12, 14, 16, 18, 20 Regelgrössen derart zu, dass durch deren Ansteuerung das Isozentrum 31 beibehalten wird. Wenn nun der Träger 5 um 90° gedreht werden soll, so dass die Röntgenröhre 7 und der Bildschichtträger 8 in die gestrichelt dargestellte Position kommen, wird über dem Steuerpult 25 der Motor 18 angesteuert, damit der Träger 5 um die Welle 6 geschwenkt wird. Die Regeleinrichtung 22 steuert dann in beschriebener Weise die weiteren Motoren 14 und 16 derart nach, dass das voreingestellte Isozentrum 31 bei der Lageänderung der Röntgenröhre bzw. des Bildschichtträgers erhalten bleibt. Die Motoren 14 und 16 bewirken, dass die Halterung 4 abgesenkt und das Teleskop herausgefahren wird. Auf entsprechende Weise wird bei anderen gewünschten Bewegungsabläufen auch die Verschiebung des Statives 1 gesteuert und der Träger 5 um die Welle 11 gedreht. Das Isozentrum 31 muss dabei nicht die geometrische Mitte zwischen Röntgenröhre 7 und Bildverstärker 8 sein, sondern kann je nach eingestellter Vergrösserung unterschiedlich liegen. Ebenso soll es mit Hilfe der Steuerung möglich sein, das Isozentrum 31 in einer beliebig liegenden Objektebene bei gleichbleibender Vergrösserung zu verschieben. Mit Hilfe eines nicht dargestellten Speichers ist es weiter möglich, die notwendigen Daten für eine bestimmte Lage des Gerätes festzuhalten und nach beliebiger Verschiebung automatisch wieder die Ausgangslage einzustellen.

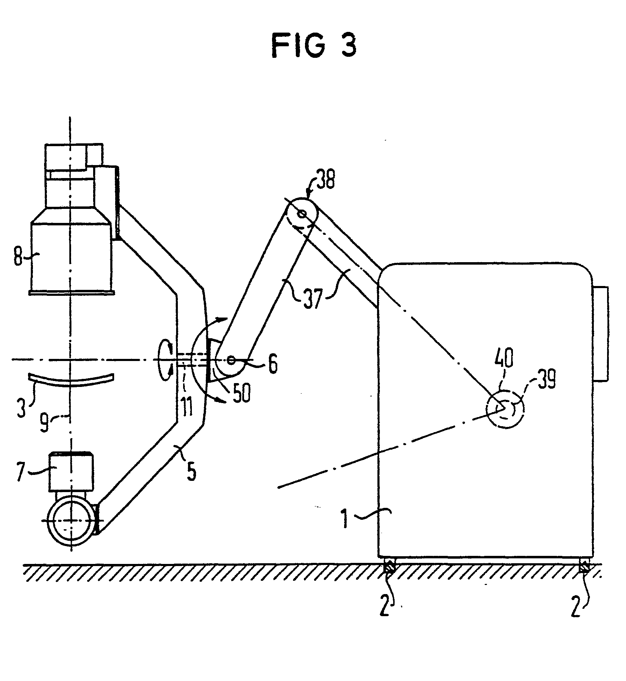

In Fig. 3 ist eine weitere Ausführungsform des erfindungsgemässen Röntgenuntersuchungsgerätes dargestellt. In diesem Ausführungsbeispiel ist der Träger 5 mittels eines zweiteiligen Gelenkarmes 37 gehalten. Zum Verstellen der beiden Teile gegeneinander ist ein Motor 38 vorgesehen. Das weitere Ende des Armes 37 ist im Stativ 1 um eine Welle 39 mittels eines Motors 40 schwenkbar. Den Motoren 38, 40 sind nicht dargestellte Lagesensoren zugeordnet, die an der Regeleinrichtung angeschlossen sind (Fig. 2). Durch die Befestigung und Bewegung des Armes 37 kann das Stativ 1 in dieser Ausführung niedrig gehalten werden.

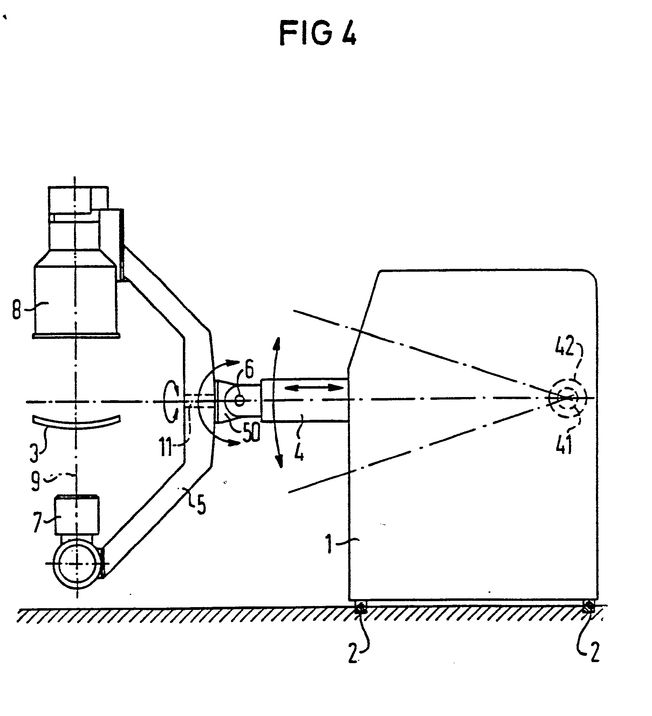

In Fig. 4 ist gezeigt, dass die teleskopartige Halterung 4 um eine Welle 41 im Stativ 1 schwenkbar gelagert ist. Als Antrieb und Steuerung werden ein Motor 42 und ein nicht dargestellter Lagesensor verwendet, die an der in Fig. 2 gezeigten Regeleinrichtung angeschlossen sind. Das Stativ 1 kann durch die Halterung 4 auch in dieser Ausführungsform niedrig gehalten werden.

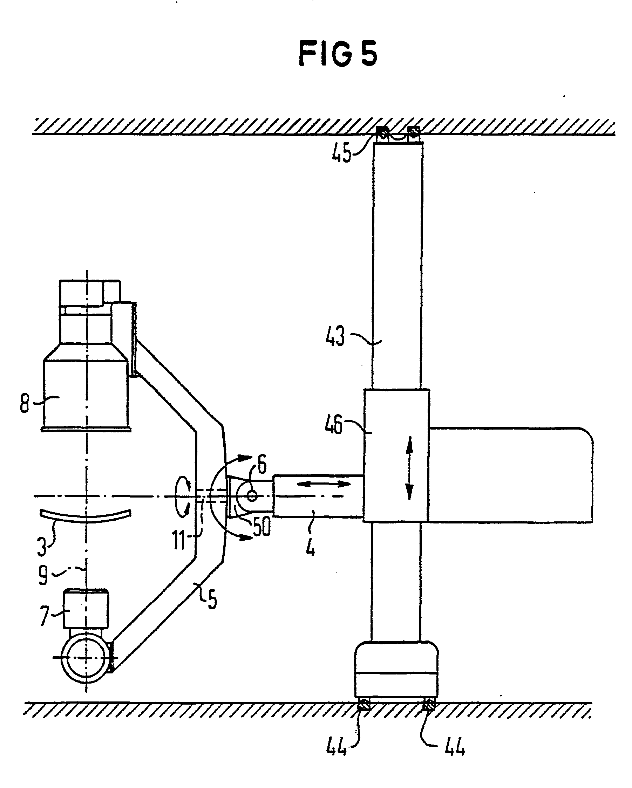

Fig. 5 zeigt schliesslich ein Röntgenuntersuchungsgerät mit einem Stativ 43, das sowohl auf Boden- als auch auf Deckenschienen 44, 45 läuft und an der ein Stativwagen 46 längsverschieblich geführt ist. Die teleskopartige Halterung 4 ist an den Stativwagen 46 fest angeordnet.

Die Bewegung des Röntgenuntersuchungsgerätes, die durch die in der Länge einstellbare Halterung 4 bzw. dem Gelenkarm 37 erfolgt, kann durch eine entsprechende Verschiebung des Statives 1, 43 ersetzt werden. Das kann erfolgen, indem in dieser Richtung bekannte und daher nicht dargestellte Schienen auf dem Boden oder an der Decke verlegt werden, auf denen das Stativ 1, 43 fährt. Vorteilhaft ist es auch möglich, das Stativ 1, 43 lediglich an der Decke aufzuhängen.

Durch das erfindungsgemässe Röntgenuntersuchungsgerät kann mit der Röntgenröhre bzw. dem Bildschichtträger ein sphärischer Bewegungsablauf dadurch vorgenommen werden, dass die Bewegungen der Geräteteile mechanisch entkoppelt sind und dass die verschiedenen Bewegungsabläufe synchron über eine Regeleinrichtung gesteuert werden. Durch die Bewegungsabläufe der Geräteteile kann auch der Träger in einer einfachen und billigen Form hergestellt werden.

## Patentansprüche

1. Röntgenuntersuchungsgerät mit einem Stativ und einer Halterung, die im Raum bzw. gegeneinander derart verstellbar sind, dass ein an der Halterung befestigter gebogener Träger, an dessen einen Ende eine Strahlungsquelle und an dessen anderen Ende ein Bildschichtträger aufeinander ausgerichtet befestigt sind, dreidimensional einstellbar ist, dass der Träger um eine erste Welle drehbar ist und um eine weitere, horizontal und senkrecht zur ersten Welle am vorderen Ende der Halterung liegende Welle schwenkbar gelagert ist, dadurch gekennzeichnet, dass der Träger (5) unverschiebbar an der Halterung (4) befestigt ist und dass für jede Verstell-, Dreh- oder Schwenkbewegung ein Antrieb (12, 14, 16, 18, 20) und ein Lagesensor (13, 15, 17, 19, 21) vorgesehen sind.

2. Röntgenuntersuchungsgerät nach Anspruch 1, dadurch gekennzeichnet, dass die Länge der Halterung (4) einstellbar ist.

3. Röntenuntersuchungsgerät nach Anspruch 1 oder 2, dadurch gekennzeichnet, dass die Halterung (4) als Teleskop ausgebildet ist.

4. Röntgenuntersuchungsgerät nach Anspruch 1 bis 3, dadurch gekennzeichnet, dass die Halterung (4) an der Säule um eine Welle (41) in vertikaler Richtung schwenkbar gelagert ist.

5. Röntgenuntersuchungsgerät nach einem der Ansprüche 1 oder 4, dadurch gekennzeichnet, dass die Halterung als ein zweiteiliger Gelenkarm (37) ausgebildet ist.

6. Röntgenuntersuchungsgerät nach einem der Ansprüche 1 bis 5, dadurch gekennzeichnet, dass der Träger (5) seitlich am vorderen Ende der Halterung (4, 37) angebracht ist.

7. Röntgenuntersuchungsgerät nach einem der Ansprüche 1 bis 6, dadurch gekennzeichnet, dass die Signale der Lagesensoren (13, 15, 17, 19, 21) auf eine Regeleinrichtung 22 gegeben sind, die bei vorgegebenem Isozentrum bei einer Lageänderung in einer Richtung Regelgrössen für die anderen Antriebe (12, 14, 16, 18, 20) derart ermittelt, dass durch deren Betätigung wieder das Isozentrum eingestellt wird.

## Claims

1. X-ray examination apparatus having a stand and a mount, which are adjustable in space or one against the other in such a way that a bent carrier attached to the mount, at one end of which carrier a radiation source is attached to be aligned with an X-ray film support attached at the other end of said carrier, can be set in three dimensions, and that the carrier can be rotated about a first shaft, and is mounted to swivel about a further shaft located at the front end of the mount horizontal and vertical to the first shaft, characterized in that the carrier (5) is attached immovably to the mount (4), and in that a drive (12, 14, 16, 18, 20) and a position sensor (13, 15, 17, 19, 21) are provided for each adjusting, rotary or swivelling motion.

2. X-ray examination apparatus according to Claim 1, characterized in that the length of the mount (4) can be set.

3. X-ray examination apparatus according to Claim 1 or 2, characterized in that the mount (4) is constructed telescopically.

4. X-ray examination apparatus according to Claim 1 to 3, characterized in that the mount (4) is mounted at the column to swivel in the vertical direction about a shaft (41).

5. X-ray examination apparatus according to one of Claims 1 or 4, characterized in that the mount is constructed as a two-piece articulated arm (37).

6. X-ray examination apparatus according to one of Claims 1 to 5, characterized in that the carrier (5) is fixed laterally at the front end of the mount (4, 37).

7. X-ray examination apparatus according to one of Claims 1 to 6, characterized in that the signals of the position sensors (13, 15, 17, 19, 21) are passed to a controller (22), which, given a change in position in one direction for a predetermined isocentre, determines controlled variables for the other drives (12, 14, 16, 18, 20) in such a way that the isocentre is reset by their actuation.

## Revendications

1. Appareil de radiodiagnostic comportant un statif et un dispositif de fixation, réglables dans l'espace ou l'un par rapport à l'autre de telle manière qu'un support coudé, qui est fixé au dispositif de fixation et sur une extrémité duquel est fixée une source de rayonnement et sur l'autre extrémité duquel est fixé un support d'une couche de formation d'images, la source de rayonnement et le support de la couche de formation d'images étant alignés l'un sur l'autre, est réglable dans trois des dimensions de sorte que le support peut tourner autour d'un premier arbre et y est tourillonné de manière à pouvoir pivoter autour d'un autre arbre horizontal, disposé sur l'extrémité avant du dispositif de fixation, en étant perpendiculaire au premier arbre, caractérisé par le fait que le support (5) est monté de façon fixe sur le dispositif de fixation (4) et qu'il est prévu, pour chaque mouvement de réglage, de rotation ou de pivotement, un dispositif d'entraînement (12, 14, 16, 18, 20) et un capteur de position (13, 15, 17, 19, 21).

2. Appareil de radiodiagnostic suivant la revendication 1, caractérisé par le fait que la longueur du dispositif de fixation (4) est réglable.

3. Appareil de radiodiagnostic suivant la revendication 1 ou 2, caractérisé par le fait que le dispositif de fixation (4) est réalisé sous la forme d'un dispositif télescopique.

4. Appareil de radiodiagnostic suivant les revendications 2 à 3, caractérisé par le fait que le dispositif de fixation (4) est tourillonné sur le statif de manière à pouvoir pivoter dans une direction verticale autour d'un arbre (41).

5. Appareil de radiodiagnostic suivant l'une des revendications 1 ou 4, caractérisé en ce que le dispositif de fixation est réalisé sous la forme d'un bras articulé (37) à deux éléments.

6. Appareil de radiodiagnostic suivant l'une des revendications 1 à 5, caractérisé par le fait que le support (5) est monté latéralement sur l'extrémité avant du dispositif de fixation (4, 37).

7. Appareil de radiodiagnostic suivant l'une des revendications 1 à 6, caractérisé par le fait que les signaux des capteurs de position (13, 15, 17, 19, 21) sont envoyés à un dispositif de régulation (22), qui, pour un barycentre prédéterminé, détermine, lors d'un changement de position dans une direction, des grandeurs de réglage pour les autres dispositifs d'entraînement (12, 14, 16, 18, 20) de telle sorte que le barycentre est à nouveau réglé grâce à l'actionnement de ces dispositifs.

FIG 1

# FIG 2

# FIG 3

# FIG 4

# FIG 5